# EUROPÄISCHE PATENTSCHRIFT

(11) **EP 3 265 467 B1**
(45) Veröffentlichungstag und Bekanntmachung des Hinweises auf die Patenterteilung: **17.10.2018**
(21) Anmeldenummer: 16706878.2
(22) Anmeldetag: 29.02.2016
(51) Int. Cl.: C07F 15/00, A61P 35/00, A61K 31/282, A61K 31/10

(54) **METALLORGANISCHE VERBINDUNG**
ORGANOMETALLIC COMPOUND
COMPOSÉ ORGANOMÉTALLIQUE

(30) Priorität: 06.03.2015 EP 15158004
(43) Veröffentlichungstag der Anmeldung: 10.01.2018
(73) Patentinhaber: Umicore AG & Co. KG, 63457 Hanau-Wolfgang (DE)
(72) Erfinder: WOERNER, Eileen, 61130 Nidderau (DE); KARCH, Ralf, 63801 Kleinostheim (DE); RIVAS-NASS, Andreas, 64625 Bensheim (DE); DOPPIU, Angelino, 63500 Seligenstadt (DE); FREY, Annika, 63457 Hanau (DE)
(86) Internationale Anmeldenummer: PCT/EP2016/054234
(87) Internationale Veröffentlichungsnummer: WO 2016/142199

(56) Entgegenhaltungen:
- EP-A1- 2 740 737

## Beschreibung

Zur Chemotherapie von Krebserkrankungen werden u.A. Cytostatika verwendet, die als Wirkstoffe (API) Komplexverbindungen des Edelmetalls Platin verwenden, wie beispielsweise Cisplatin, Carboplatin oder Oxaliplatin.
Eine, beispielsweise im Herstellprozess von Carboplatin, mögliche Zwischenstufe ist der Cis-Diammino-diaquo-Platin-Komplex cis-[NH₃)₂Pt(H₂O)₂]²⁺. Da dieser Komplex bei nicht vollständiger Umsetzung im Produkt Carboplatin als Verunreinigung auftreten könnte, wird dieser Komplex mit einer Höchstgrenze im Produkt spezifiziert.
Zur Analyse der Wirkstoffe in der Qualitätskontrolle durch HPLC (HochdruckFlüssigkeitschromatographie) ist ein Analysenstandard erforderlich. cis-Diamminodinitratoplatin cis-[(NH₃)₂Pt(NO₃)₂], CAS 41575-87-5) ist der bisher gängige Precursor für den Cis-Diammino-diaquo-Platin-Komplex in Carboplatin.
Bei dem gängigen Standard cis-[(NH₃)₂Pt(NO₃)₂] handelt es sich um ein Salz, das beim Lösen in Wasser zu dem zweifach positiv geladenen Cis-Diammino-diaquo-Platin-Komplex cis-[(NH₃)₂Pt(H₂O)₂]²⁺ und zwei Nitrat-Anionen NO₃⁻ dissoziiert.
Bei der Verbindung cis-[(NH₃)₂Pt(NO₃)₂] handelt es sich um einen trockenen Feststoff. Es ist bekannt, dass trockene Verbindungen von Ammoniak und Nitrat, vor allem bei Energiezufuhr z.B. bei Stoß oder Schlag zur spontanen exothermen Zersetzung oder sogar Explosion neigen können. Dieser Standard ist somit sicherheitstechnisch bedenklich und sollte ohne aufwändige Sicherheitsvorkehrung nicht gehandhabt werden.

EP-A-2740737 beschreibt das Dihydrat von 1,2-Cyclohexandiaminplatin(II)bis-(4-methylbenzolsulfonat) und ein Verfahren zu dessen Herstellung.

Bartocci et al., Inorganica Chimica Acta 53, (1981), L157-L159 zeigt die Verbindung cis-[Pt(NH₃)₂(H₂O)₂]SO₄, deren Herstellung und Polymere. Allgemein werden diese Polymere als Pt-Blues beschrieben und werden immer beschrieben als di- oder Polymere des cis-[Pt(NH₃)₂(H₂O)]²⁺ Komplexes.
Diese werden in Bartocci et al. Mit der folgenden Formel beschrieben:

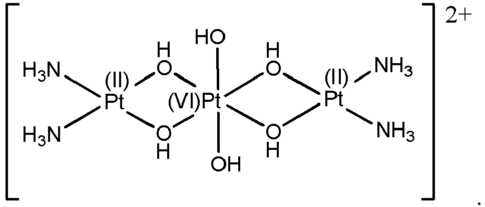

Appleton et al., Inorganica Chimica Acta 64, (1982), L229-L233, Appleton et al., Inorganic Chemistry Vol. 23, No. 22, (1984), 3514-3521, Flynn et al., J. Inorg. Nucl. Chem. 39, (1977), 437-439 and Lippert et al., Inorganic Chemistry Vol. 16, No. 6, (1977), 1525-1529 beschreiben mehrere Diammino-diaquo-komplexe sowie deren Polymerisation, wobei es sich hierbei um mehrere nicht näher definierte Polymere handelt, deren Struktur z.T. nicht vollständig geklärt ist und die als "platinum blues" bezeichnet werden.

Es war daher die Aufgabe, eine neue Verbindung bereitzustellen, die in wässriger Lösung den gleichen Cis-Diammino-diaquo-Platin-Komplex cis-[NH₃)₂Pt(H₂O)₂]²⁺ ergibt wie die Lösung des derzeit üblichen Standards, die als Feststoff isolierbar und über längere Zeit stabil ist. Außerdem muss diese Verbindung zuverlässig analysierbar und in Wasser leicht löslich sein und nicht zu gefährlichen Zersetzungsreaktionen neigen.

Diese Aufgabe wird gelöst durch die Verbindung cis-[(NH₃)₂Pt(H₂O)₂](O-Tos)₂, cis-(diammino-diaquo)platin di-(p-toluolsulfonat) der Formel 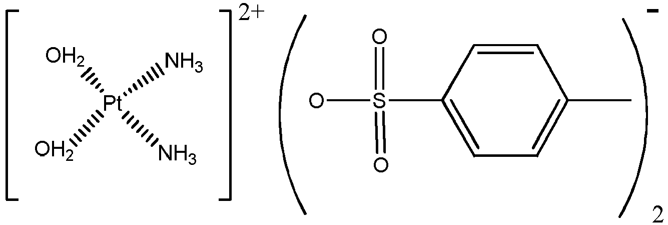 und dessen Polymere. Diese Verbindung kann in hoher Reinheit und mit einem niedrigen Silbergehalt von 3 - 70 ppm erhalten werden. Die Polymere dürften eine ähnliche Struktur aufweisen wie von Bartocci et al., Inorganica Chimica Acta 53, (1981), L157-L159 (siehe oben) beschrieben. Die Stereochemie des Kations kann etwas deutlicher auch in dieser Form dargestellt werden:

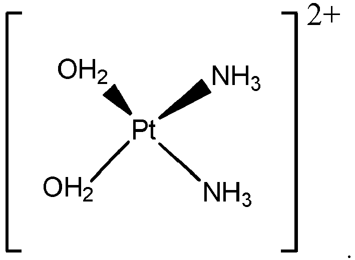

Das Produkt kann erhalten werden durch ein Verfahren zur Herstellung dieser Verbindung mit den folgenden Schritten:
a) Bereitstellen einer bestimmten Menge eines cis-diamminodihalogenido Pt (II)-Komplexes in wässriger Lösung bzw. Suspension;
b) Umsetzen dieses cis-diamminodihalogenido Pt (II) Komplexes mit der 1,8 - bis 2,2-fachen molaren Menge (bezogen auf den cis-diamminodihalogenido Pt (II) Komplex) an Silber-p-toluolsulfonat bei einer Temperatur von weniger als 100°C unter Rühren;
c) Optionale Zugabe eines Alkali- oder Erdalkalihalogenids zur Fällung überschüssiger Silberionen;
d) Einfache oder mehrfache Filtration zur Abtrennung von unlöslichen Silberhalogeniden;
e) Fällung des Reaktionsproduktes;
f) Abfiltrieren des Reaktionsproduktes, um das feste Reaktionsprodukt und das Filtrat zu erhalten, sowie optionales Waschen des Reaktionsproduktes.

### Kurze Beschreibung der Erfindung

1. Die Verbindung cis-[(NH₃)₂Pt(H₂O)₂](O-Tos)₂, cis-(diammino-diaquo)platin di-(p-toluolsulfonat) der Formel 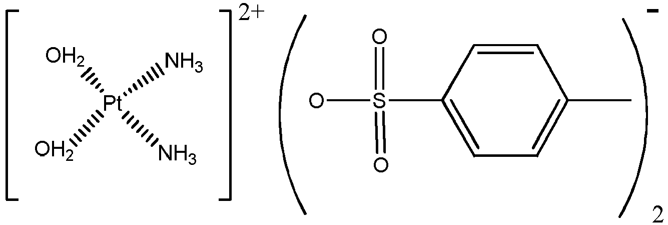 und dessen Polymere.
2. Verfahren zur Herstellung der Verbindung nach Punkt 1, gekennzeichnet durch die folgenden Schritte:
   a) Bereitstellen einer bestimmten Menge eines cis-diamminodihalogenido Pt (II)-Komplexes in wäßriger Lösung;
   b) Umsetzen dieses cis-diamminodihalogenido Pt (II) Komplexes mit der 1,8 - bis 2,2-fachen molaren Menge (bezogen auf den cis-diamminodihalogenido Pt (II) Komplex) an Silber-p-toluolsulfonat bei einer Temperatur von weniger als 100°C unter Rühren;
   c) Optionale Zugabe eines Alkali- oder Erdalkalihalogenids zur Fällung überschüssiger Silberionen;
   d) Einfache oder mehrfache Filtration zur Abtrennung von unlöslichen Silberhalogeniden;
   e) Fällung bis des Reaktionsproduktes;
   f) Abfiltrieren des Reaktionsproduktes, um das feste Reaktionsprodukt und das Filtrat zu erhalten und optionales Waschen des Reaktionsproduktes.
3. Verfahren nach Punkt 2, wobei die Fällung in Schritt e) durch Abdestillieren von Wasser unter reduziertem Druck bei einer Temperatur von unter 50°C, durch Zugabe eines Fällungsmittels oder deren Kombinationen bewirkt wird.
4. Verfahren nach Punkt 3, wobei etwa 75% bis etwa 65% des Wassers abdestilliert wird.
5. Verfahren nach Punkt 3, wobei das Fällungsmittel ein organisches Lösemittel ist, insbesondere Ethanol oder Aceton.
6. Verfahren nach einem oder mehreren der Punkte 2 bis 5, wobei Schritt c) einen Fällungsschritt zur Fällung überschüssiger Erdalkaliionen umfasst.
7. Verfahren nach einem oder mehreren der Punkte 2 bis 6, wobei das Silber-p-toluolsulfonat gegenüber dem cis-diamminodihalogenido Pt(II)-Komplex in der zweifachen molaren Menge eingesetzt wird.
8. Verfahren nach einem oder mehreren der Punkte 2 bis 7, wobei Schritt a), die Bereitstellung des cis-diamminodihalogenido Pt(II)-Komplexes, durch Reaktion von K₂PtCl₄ mit Kaliumiodid und Ammoniak erfolgt.
9. Verfahren nach einem oder mehreren der Punkte 2 bis 8, enthaltend den weiteren Schritt g), in welchem das Filtrat auf eine Temperatur zwischen 0°C und 10°C gekühlt und ausgefallenes Reaktionsprodukt abfiltriert wird.
10. Verfahren nach einem oder mehreren der Punkte 2 bis 9, wobei Schritt f) oder g) ein weiterer Schritt zur Kristallisation des Endproduktes, insbesondere mindestens ein Schritt ausgewählt aus der Gruppe bestehend aus Umkristallisation, einer Wiederholung der Schritte f), g) und deren Kombinationen, folgt.
11. Verfahren nach einem oder mehreren der Punkte 2 bis 10, wobei in Schritt e) vor dem Fällen der pH-Wert durch Zugabe einer Säure auf einen pH-Wert von 1,5 oder niedriger, vorteilhaft von 1 oder niedriger eingestellt wird.
12. Verfahren nach Punkt 11, wobei eine organische oder anorganische Säure eingesetzt wird.
13. Verfahren nach einem oder mehreren der Punkte 11 bis 12, wobei die anorganische Säure ausgewählt ist aus Salzsäure (HCl) oder Schwefelsäure (H₂SO₄), Salpetersäure (HNO₃).
14. Verfahren nach einem oder mehreren der Punkte 11 bis 12, wobei die organische Säure ausgewählt ist aus Ameisensäure, Essigsäure, p-Toluolsulfonsäure, p-Toluolsulfonsäuremonohydrat, Weinsäure, Zitronensäure, Cyclobutandicarbonsäure.
15. Verfahren nach einem oder mehreren der Punkte 2 bis 14, wobei das abfiltrierte Reaktionsprodukt aus den Schritten f) oder g) unter Sauerstoffausschluß getrocknet wird.
16. Verfahren nach einem oder mehreren der Punkte 2 bis 15, wobei das Verfahren unter Lichtausschluß, Sauerstoffausschluß oder deren Kombinationen durchgeführt wird.
17. Verfahren nach einem oder mehreren der Punkte 2 bis 16, wobei als cis-diamminodihalogenido Pt(II)-Komplex ein cis-diamminodiiodo Pt(II)-Komplex, cis-diamminodichloro Pt (II)-Komplexe oder cis-diamminodibromo Pt (II)-Komplexe eingesetzt wird, insbesondere cis-diamminodiiodo Pt (II)-Komplexe, insbesondere Platin-cis-Diammindiiodid
18. Verfahren zur Herstellung einer Zusammensetzung enthaltend Wasser, cis-(diammino-diaquo)platin di-(p-toluolsulfonat) und optionale weiteren Substanzen, wobei cis-(diammino-diaquo)platin di-(p-toluolsulfonat) nach einem oder mehreren der Punkte 2 bis 17 hergestellt und in Wasser oder einer wässrigen Lösung aufgelöst wird.
19. Verbindung cis-(diammino-diaquo)platin di-(p-toluolsulfonat) nach Punkt 1 mit einem Silbergehalt von 3 ppm bis 70 ppm.
20. cis-(diammino-diaquo)platin di-(p-toluolsulfonat) nach Punkt 1, erhältlich nach einem oder mehreren der Punkte 2 bis 17.
21. Verwendung von cis-(diammino-diaquo)platin bis-(p-toluolsulfonat) nach Punkt 1 oder dessen Zubereitungen als Analysenstandard.
22. Analysenkit enthaltend cis-(diammino-diaquo)platin di-(p-toluolsulfonat) nach Punkt 1 oder cis-(diammino-diaquo)platin bis-(p-toluolsulfonat) erhältlich nach einem oder mehreren der Punkte 2 bis 17 oder deren Zubereitungen.
23. Verwendung der Verbindung nach Punkt 1 als Precursor für die Synthese von cytostatisch aktiven Platinkomplexen.
24. Verbindung nach Punkt 1 zur Verwendung als Aktivsubstanz in Cytostatika.

### Detaillierte Beschreibung der Erfindung

Als cis-diamminodihalogenido Pt (II)-Komplex können vorteilhaft cis-diamminodiiodo Pt (II)-Komplexe, cis-diamminodichloro Pt (II)-Komplexe oder cis-diamminodibromo Pt (II)-Komplexe eingesetzt werden, insbesondere cis-diamminodiiodo Pt (II)-Komplexe. Insbesondere geeignet ist Platin-cis-Diammindiiodid, was durch Reaktion von K₂PtCl₄ mit Kaliumiodid und Ammoniak erhalten werden kann (Inorganic Syntheses (1989), 25, 98-100)
Durch Reaktion des cis-diamminodihalogenido Pt(II)-Komplexes, insbesondere cis-diamminodiiodo Pt (II)-Komplexes, mit der 1,8 - bis 2,2-fachen molaren Menge (bezogen auf den cis-diamminodihalogenido Pt(II)-Komplex bzw. cis-diamminodiiodo Pt (II) Komplex), oder der 1,9 bis 2,1-fachen molaren Menge, insbesondere der doppelten molaren Menge an Silber-p-toluolsulfonat in wäßriger Lösung, wird das Produkt erhalten.

Die Reaktion kann so geführt werden, daß eine wäßrige Lösung oder Suspension des cis-diamminodihalogenido Pt(II)-Komplexes, insbesondere des cis-diamminodiiodo Pt (II)-Komplexes, insbesondere Platin-cis-Diammindiiodid, vorgelegt und mit festem Silber-p-toluolsulfonat versetzt wird.

Alternativ dazu kann das Silber-p-toluolsulfonat auch in situ im Reaktionsgemisch hergestellt werden, beispielsweise durch Reaktion von Silberoxid und Toluolsulfonsäure. Diese kann im Reaktionsgefäß erfolgen und der cis-diamminodihalogenido Pt(II)-Komplex anschließend zugegeben werden, wie zum Beispiel als Feststoff, Lösung oder Suspension. Die in situ Herstellung des Silber-p-toluolsulfonats kann aber auch in einem zweiten Reaktionsgefäß erfolgen und dann zum vorgelegten cis-diamminodihalogenido Pt(II)-Komplex zugegeben werden.

Im Falle einer Suspension löst sich der ungelöste cis-diamminodihalogenido Pt(II)-Komplex bzw. cis-diamminodiiodo Pt (II)-Komplex im Zuge der Reaktion allmählich auf.
Die Konzentration des cis-diamminodihalogenido Pt(II)-Komplexes bzw. cis-diamminodiiodo Pt (II)-Komplexes bzw. dessen Verhältnis zum Lösemittel Wasser ist im Allgemeinen unkritisch und richtet sich nach den vorhandenen Produktionsanlagen. Für eine zügige Reaktion sollte keine zu große Lösemittelmenge gewählt werden, aber wegen der begrenzten Wasserlöslichkeit dieses Eduktes aber auch keine zu geringe Menge. Bewährt haben sich Gewichtsverhältnisse von cis-diamminodihalogenido Pt(II)-Komplex bzw. cis-diamminodiiodo Pt (II)-Komplex zu Wasser im Bereich von 1:100 bis 100:1, oder von 5:100 bis 20:100, oder 8:100 bis 10:100.
Die Reaktion wird unter Rühren bei unter 100°C durchgeführt. Bewährt haben sich Temperaturen im Bereich von 40°C bis 90°C, oder von 50°C bis 80°C oder von 60°C bis 75°C. Die Reaktionzeit beträgt meist etwa 2 bis etwa 24 Stunden, insbesondere etwa 4 bis etwa 16 Stunden oder etwa 6 bis etwa 12 Stunden.

Wird das Silber-p-toluolsulfonat in Mengen von weniger als der zweifachen molaren Menge (bezogen auf den cis-diamminodihalogenido Pt(II)-Komplex bzw. cis-diamminodiiodo Pt (II) Komplex) eingesetzt, so muß entweder eine ausreichend hohe Wassermenge verwendet werden, um nicht umgesetzten cis-diamminodihalogenido Pt(II)-Komplex bei Reaktionsende in wäßriger Lösung zu halten oder aber das Reaktionsprodukt ausreichend lang gewaschen oder umkristallisiert werden. Dies wird mit zunehmender Menge des eingesetzten Silber-p-toluolsulfonats unkritischer, ab einer zweifachen molaren Menge von Silber-p-toluolsulfonat tritt im Allgemeinen ein im Wesentlichen quantitativer Umsatz ein, so dass die Menge an nicht umgesetztem cis-diamminodihalogenido Pt(II)-Komplex bei der Reaktionsführung nicht mehr berücksichtigt werden muss.

Wird das Silber-p-toluolsulfonat in Mengen von mehr als der zweifachen molaren Menge (bezogen auf den cis-diamminodihalogenido Pt(II)-Komplex) eingesetzt, so ist ein Zusatz von Alkali- oder Erdalkalihalogeniden empfehlenswert, um überschüssiges Silber in Form von Silberhalogenid auszufällen.
Das Alkali- oder Erdalkalihalogenid kann ausgewählt sein aus der Gruppe bestehend aus den Chloriden, Bromiden, Iodiden oder Fluoriden von Natrium, Kalium, Lithium, Rubidium, Cäsium, Beryllium, Magnesium, Calcium, Strontium, Barium und deren Mischungen. Es ist empfehlenswert, gut wasserlösliche Verbindungen auszuwählen, da auf diese Weise möglichst viel Alkali- oder Erdalkalihalogenid in einem möglichst niedrigen Wasservolumen gelöst werden kann. Dies sind zum Beispiel Chloride oder Bromide des Natriums oder Kaliums, insbesondere Natriumchlorid oder Kaliumchlorid.
Falls Erdalkalihalogenide eingesetzt werden, können überschüssige Erdalkalimetallionen durch Zusatz entsprechender Fällungsmittel wiederum ebenfalls ausgefällt werden, beispielsweise durch Zusatz von Kohlensäure um überschüssiges Calciumchlorid als Calciumcarbonat zu fällen oder Zusatz von Schwefelsäure zur Fällung von überschüssigem Barium oder Strontium. Meist sind aber die Alkalimetallhalogenide, insbesondere Chloride oder Bromide des Natriums oder Kaliums, insbesondere Natriumchlorid oder Kaliumchlorid vorzuziehen.

Anschließend wird das Reaktionsgemisch filtriert bis eine klare Lösung erhalten wird. Hierzu hat sich eine gegebenenfalls wiederholte Filtration über eine Glasfritte, insbesondere eine G4-Glasfritte, bewährt.

Aus der klaren Lösung wird dann das Reaktionsprodukt gefällt. In einer Ausgestaltung der Erfindung kann dies durch Zusatz von Fällungsmitteln bewirkt werden. Hierzu sind insbesondere polare, organische Lösemittel geeignet, in welchen das Reaktionsprodukt unlöslich oder schlecht löslich ist. Diese dürfen nicht mit dem Reaktionsprodukt reagieren, wie beispielsweise einen Ligandenaustausch eingehen und müssen ausserdem hinreichend wasserlöslich sein. Gut geeignet sind beispielsweise Aceton oder Ethanol.

Alternativ hierzu kann Wasser aus dem Reaktionsgemisch entfernt, insbesondere abdestilliert werden, bis das Reaktionsprodukt ausfällt. Hierzu wird insbesondere unter reduziertem Druck bei einer Temperatur von unter 50°C gearbeitet, was bequem mit handelsüblichen Rotationsverdampfern durchgeführt werden kann. Gute Ergebnisse lassen sich durch die Entfernung von etwa 75 % bis etwa 65% des Wassers erzielen. Das Reaktionsprodukt kann dann abfiltriert und optional gewaschen werden, wobei als Waschmedium Wasser, kaltes Wasser sowie organische Lösungsmittel wie z.B. Ethanol oder Aceton geeignet sind.

Wird vor der Fällung des Reaktionsproduktes der pH-Wert durch Säurezugabe auf einen Wert von 1,5 oder niedriger, vorteilhaft von 1 oder niedriger eingestellt, so wird monomeres Reaktionsprodukt cis-(diammino-diaquo)platin di-(p-toluolsulfonat) erhalten. Wird auf die Einstellung des pH-Wertes verzichtet, so erhält man auch polymeres cis-(diammino-diaquo)platin di-(p-toluolsulfonat). An die zur Einstellung des pH-Wertes verwendeten Säuren ist die Anforderung zu stellen, dass sie keinen Ligandenaustausch am Platinatom des Reaktionsproduktes eingehen, da ansonsten nicht das erwünschte Produkt erhalten wird. Hierzu können organische oder anorganische Säuren verwendet werden, wie beispielsweise Salzsäure (HCl), Schwefelsäure (H₂SO₄), Salpetersäure (HNO₃), Ameisensäure, Essigsäure, p-Toluolsulfonsäure, p-Toluolsulfonsäuremonohydrat, Weinsäure, Zitronensäure, Cyclobutandicarbonsäure oder deren Kombinationen.

Zur Optimierung der Ausbeute kann das Filtrat auf eine Temperatur zwischen 0°C und 10°C gekühlt werden, wobei weiteres Reaktionsprodukt ausfällt und abfiltriert werden kann. Auch hier hat sich die Filtration über eine G4-Glasfritte bewährt.
Anschließend können weitere Schritte zur Kristallisation des Endproduktes durchgeführt werden. Hierzu kann aus dem Filtrat erneut Wasser entfernt und filtriert und/oder gegebenenfalls erneut gekühlt und filtriert werden.
Auch eine Umkristallisation aus Wasser ist möglich, wobei nach dem erneuten Auflösen des Reaktionsproduktes erneut gefällt werden muss, entweder durch Einengen der Lösung, Kühlen der Lösung oder deren Kombinationen, wobei ausfallendes Reaktionsprodukt wieder abfiltriert und wie oben beschrieben gegebenenfalls gewaschen wird.

Das erhaltene cis-(diammino-diaquo-platin) di-(p-toluolsulfonat) kann nach dem Filtrieren getrocknet werden. Dies wird vorteilhaft unter Inertgas in reduziertem Druck durchgeführt.

Insbesondere ist es vorteilhaft, wenn Sauerstoff im Verlauf der gesamten Reaktion so weit wie möglich ausgeschlossen wird, wozu beispielsweise die zur Herstellung verwendeten Apparaturen evakuiert und mit Schutzgas neu befüllt sowie Lösemittel und Ausgangsstoffe entgast und unter Schutzgas aufbewahrt werden. Als Schutzgas geeignet sind insbesondere Stickstoff und Argon. Zusätzlich ist auch Lichtausschluß ratsam.
Die Verbindung cis-(diammino-diaquo-platin) bis-(p-toluolsulfonat), welche nach dem oben beschriebenen Verfahren erhalten werden kann, ist als Analysenstandard bei der Herstellung bzw. Analyse und Qualitätskontrolle platinhaltiger Cytostatika wie Cisplatin, Carboplatin oder Oxaliplatin geeignet.

Die vorliegende Patentanmeldung betrifft daher auch die Verwendung von cis-(diammino-diaquo-platin) bis-(p-toluolsulfonat) und dessen Zubereitungen als Analysenstandard .
Die Analyse erfolgt im Allgemeinen durch HochdruckFlüssigkeitschromatographie (HPLC). Daher ist das cis-(diammino-diaquo-platin) bis-(p-toluolsulfonat) vor der Verwendung in einem Lösemittel, insbesondere Wasser, aufzulösen. Daher betrifft die vorliegende Patentanmeldung auch ein Verfahren zur Herstellung einer Zusammensetzung enthaltend Wasser, cis-(diammino-diaquo-platin) di-(p-toluolsulfonat) und optionale weiteren Substanzen, wobei cis-(diammino-diaquo-platin) di-(p-toluolsulfonat) in Wasser oder einer wässrigen Lösung aufgelöst wird.
Als weitere Substanzen können beispielsweise Hilfsstoffe, Stabilisatoren oder bekannte Nebenprodukt oder Edukte eingesetzt werden, welche bei der Herstellung platinhaltiger Cytostatika wie Carboplatin oder Oxaliplatin verwendet werden, also zum Beispiel Cyclobutandicarbonsäure, Cyclohexandiamin oder auch Ethandisäure.

Die Verbindung cis-(diammino-diaquo-platin) di-(p-toluolsulfonat) sowie deren Zubereitungen können auch in Analysenkits zur Analyse oder Bestimmung platinhaltiger Cytostatika produziert, vertrieben und genutzt werden. Daher betrifft die vorliegende Patentanmeldung auch einen Analysenkit zur Analyse platinhaltiger Cytostatika, enthaltend cis-(diammino-diaquo-platin) di-(p-toluolsulfonat) sowie Zubereitungen enthaltend diese Verbindung.

Zur Herstellung von vielen platinhaltigen cytostatisch aktiven Platinkomplexen (wie z.B. Carboplatin oder cis-Platin) tritt der Cis-Diammino-diaquo-Platin-Komplex cis-[(NH₃)₂Pt(H₂O)₂]²⁺ als Zwischenprodukt auf, wobei cis-(diammino-diaquo)platin di-(p-toluolsulfonat), (cis-[(NH₃)₂Pt(H₂O)₂](O-Tos)₂) und dessen Polymere als Quelle für den Cis-Diammino-diaquo-Platin-Komplex cis-[NH₃)₂Pt(H₂O)₂]²⁺ dienen können. Daher betrifft die vorliegende Patentanmeldung auch die Verwendung von cis-(diammino-diaquo)platin di-(p-toluolsulfonat), (cis-[(NH₃)₂Pt(H₂O)₂](O-Tos)₂) und dessen Polymeren als Precursor für die Synthese von cytostatisch aktiven Platinkomplexen.

Eine weitere Nutzungsmöglichkeit für die Verbindung cis-(diammino-diaquo)platin di-(p-toluolsulfonat), (cis-[(NH₃)₂Pt(H₂O)₂](O-Tos)₂) und dessen Polymeren ist der Einsatz als Aktivsubstanz, also als pharmazeutischer Wirkstoff (API, Active Pharmaceutical Ingredient) und somit als Bestandteil von Arzneimitteln (Pharmazeutika)mit cytostatischer Wirkung, so genannten Cytostatika. Daher betrifft die vorliegende Patentanmeldung auch die cis-(diammino-diaquo)platin di-(p-toluolsulfonat), (cis-[(NH₃)₂Pt(H₂O)₂](O-Tos)₂) und dessen Polymeren zur Verwendung als Aktivsubstanz in Cytostatika sowie Arzneimittel, enthaltend von cis-(diammino-diaquo)platin di-(p-toluolsulfonat), (cis-[(NH₃)₂Pt(H₂O)₂](O-Tos)₂) und dessen Polymeren als pharmazeutisch aktiven Wirkstoff (API).

### Beispiele:

### Beispiel 1

Ein 250 ml-Dreihalskolben wurde für 15 Minuten mit einem Argonstrom von 10 L/h gespült und so inertisiert. Danach wurde der Strom auf 5 L/h reduziert und 7,5 g cis-Pt(NH₃)₂I₂ vorgelegt und mit 100 ml entgastem, destilliertem Wasser versetzt und gerührt. Unter Rühren wurden 8,67 g Silbertosylat mittels eines Pulvertrichters im Argon-Gegenstrom zugegeben und mit 50 ml entgastem, destilliertem Wasser die Einwaageschale und der Pulvertrichter nachgespült. Das Reaktionsgemisch wurde auf 70°C erwärmt und für 6 Stunden bei 70°C gerührt. Anschließend wurde die Wärmezufuhr unterbrochen und etwa 16 Stunden unter Rühren die Abkühlung auf Raumtemperatur gestattet.

Unter Argonatmosphäre wurde über eine Umkehrfritte G4 abfiltriert und der Filterkuchen zweimal mit 5 ml entgastem, destilliertem Wasser gewaschen. Durch Zugabe von ca. 3 ml p-Toluolsulfonsäuremonohydrat wurde der pH-Wert auf 1 eingestellt und bei einer Temperatur von 28°C und einem Druck von 30 mbar 115 ml Wasser abdestilliert, wobei ein kristalliner Niederschlag ausfiel. Dieser wurde unter Argonatmosphäre über eine Umkehrfritte G4 abfiltriert und im Argonstrom getrocknet. Das Filtrat wurde für 16 Stunden im Kühlschrank auf ca. 7°C gekühlt, wobei erneut ein kristalliner Niederschlag ausfiel. Dieser wurde ebenso abfiltriert und getrocknet.
Elementaranalyse: Pt 31,77%, C 27,3%, H 4,4%, N 5%, S 11,20%, Ag 10 ppm, I 25 ppm. (Theorie: Pt 32,11%, C 27,68%, H 3,98%, N 4,61%, S 10,56%, Ag 0 ppm, I 0 ppm)
NMR (¹⁹⁵Pt): δ = -1573,47 (s)

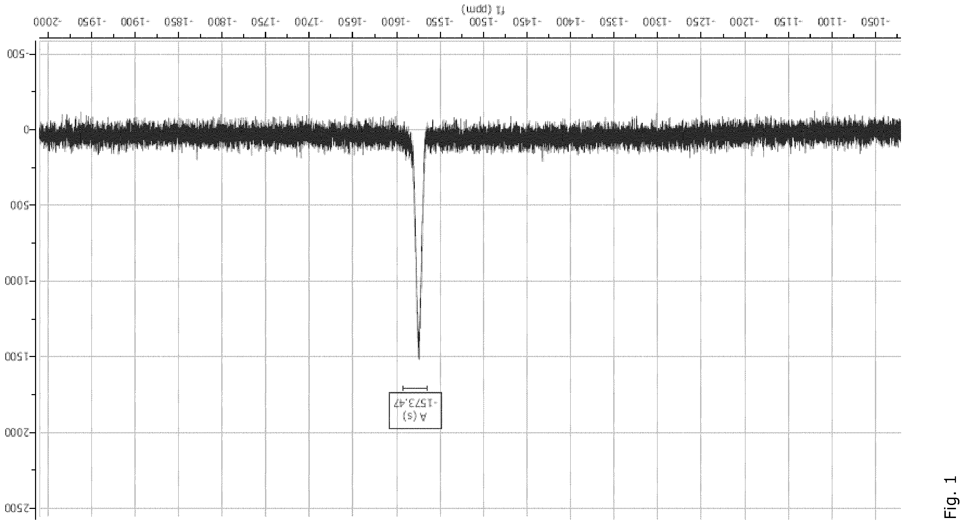

Figur 1 ist eine bildliche Darstellung des ¹⁹⁵Pt-NMR-Spektrums.

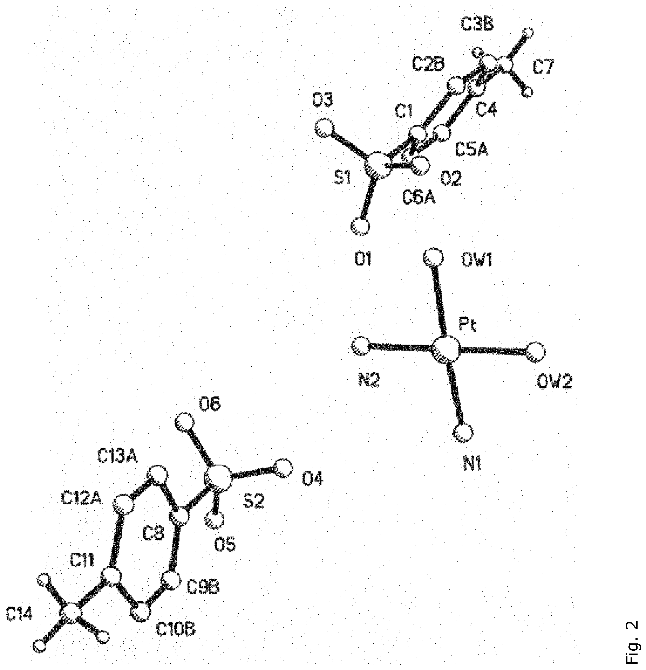

Figur 2 zeigt die Struktur der Verbindung, ermittelt durch Einkristall-Röntgenstrukturanalyse.

Strukturdaten aus der Röntgen-Einkristall-Strukturanalyse:

| | | | |
|---|---|---|---|
| Summenformel | C₁₄H₂₄N₂O₈PtS₂ | | |
| Meßtemperatur | 293 K | | |
| Wellenlänge | 71,073 pm (MοKα, Graphitmonochromator) | | |
| Kristallsystem | monoklin | | |
| Raumgruppe | P2₁/n (Nr. 14) | | |
| Gitterkonstanten | a= 616,50 (10) pm | | α= 90 ° |
| | b=1380,3(2) pm | | β= 92,758(9) ° |
| | c=2386,0(3) pm | | γ= 90 ° |
| Elementarzellenvolumen | 2028,0(5)·10⁶ pm³, Z=4 | | |
| Θ-Bereich | 2,26 - 25,0 ° | | |
| Indexbereich | h -1/7 | k -1/16 | I -28/28 |

Die Ausbeute liegt bei ca. 62% (bezogen auf eingesetztes Platin).

### Beispiel 2

In einem mit Argon inertisierten 250 ml-Dreihalskolben werden 11,1 g cis-Pt(NH₃)₂I₂ vorgelegt und mit 120 ml entgastem, destilliertem Wasser versetzt und gerührt. Unter Rühren wurden 12,85 g Silbertosylat zugegeben und mit 20 ml destilliertem Wasser die Einwaageschale und der Pulvertrichter nachgespült. Das Reaktionsgemisch wurde auf 70°C erwärmt und für 6 Stunden bei 70°C gerührt. Anschließend wurde die Wärmezufuhr unterbrochen und etwa 16 Stunden unter Rühren die Abkühlung auf Raumtemperatur gestattet.
Es wurde über eine Fritte G4 abfiltriert und der Filterkuchen zweimal mit 5 ml destilliertem Wasser gewaschen.

Aus der resultierenden Lösung wurde bei einer Temperatur von 31°C und einem Druck von 15 mbar bis zur Trockne Wasser abdestilliert, wobei ein kristalliner Niederschlag ausfiel. Dieser wurde nochmals im Argonstrom unter reduziertem Druck getrocknet.

Das Produkt besitzt eine grau blaue Färbung, was laut Literatur (u.A. Bartocci et al. s. oben) auf eine polymere Spezies hinweist.
Ausbeute: 95,1%
Elementaranalyse: Pt 31,96%, C 27,3%, H 4,3%, N 4,7%, S 11,85%, Ag 8 ppm, I 15 ppm. (Theorie für das Monomer: Pt 32,11%, C 27,68%, H 3,98%, N 4,61%, S 10,56%, Ag 0 ppm, I 0 ppm)

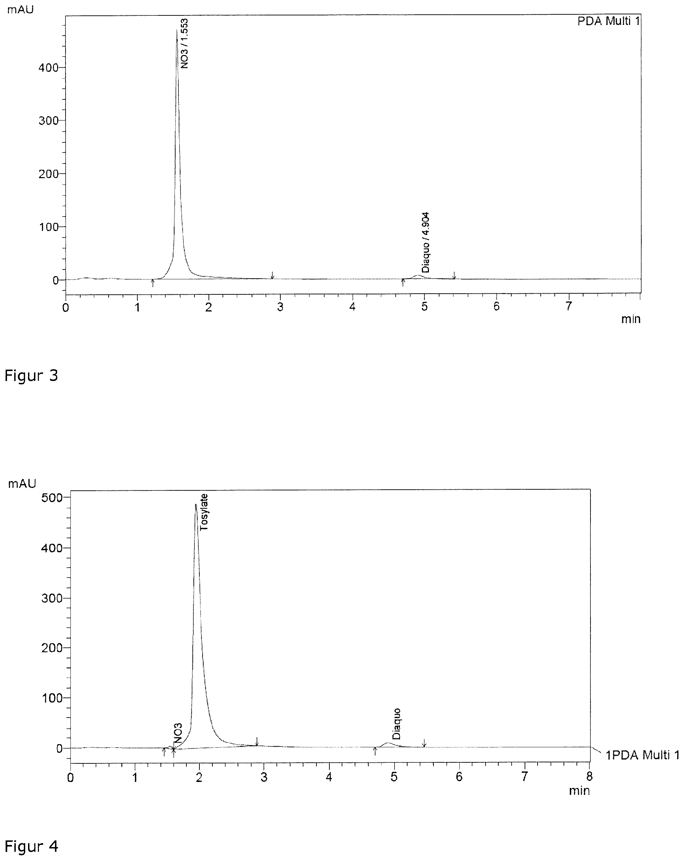

Verwendung von cis-(diammino-diaquo)platin di-(p-toluolsulfonat) als Analysenstandard
Figur 3 zeigt ein HPLC-Chromatogramm von cis-Diamminodinitratoplatin cis-[NH₃)₂Pt(NO₃)₂], CAS 41575-87-5). Gut zu erkennen ist der starke Peak bei einer Retentionszeit von 1,553 Minuten, der vom Nitration herrührt, und den schwächeren Peak bei einer Retentionszeit von 4,904 Minuten, der die Anwesenheit des cis-diammin-Pt-diaquokomplexes anzeigt.

Figur 4 zeigt ein HPLC-Chromatogramm von cis-(diammino-diaquo)platin di-(p-toluolsulfonat), cis-[(NH₃)₂Pt(H₂O)₂](O-Tos)₂. Der Nitrat-Peak bei einer Retentionszeit von 1,553 Minuten ist nur sehr schwach zu erkennen, der schwächeren Peak des cis-diammin-Pt-diaquokomplexes tritt ebenfalls auf bei einer Retentionszeit von 4,903 Minuten. Ein starker Peak bei einer Retentionszeit von 1,941 Minuten wird vom Tosylation verursacht.

Es ist somit festzustellen, daß die Verbindung gemäß der Erfindung als Analysenstandard geeignet ist, da der erwünschte cis-diammin-Ptdiaquokomplex bereitgestellt wird.

## Patentansprüche

1. Die Verbindung cis-[(NH₃)₂Pt(H₂O)₂](O-Tos)₂, cis-(diammino-diaquo)platin di-(p-toluolsulfonat) der Formel 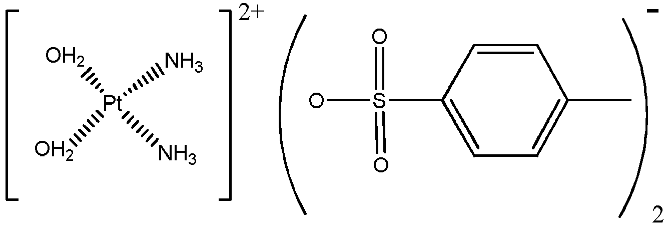 und dessen Polymere.

2. Verfahren zur Herstellung der Verbindung nach Anspruch 1, **gekennzeichnet durch** die folgenden Schritte:
a) Bereitstellen einer bestimmten Menge eines cis-diamminodihalogenido Pt (II)-Komplexes in wäßriger Lösung;
b) Umsetzen dieses cis-diamminodihalogenido Pt (II) Komplexes mit der 1,8 - bis 2,2-fachen molaren Menge (bezogen auf den cis-diamminodihalogenido Pt (II) Komplex) an Silber-p-toluolsulfonat bei einer Temperatur von weniger als 100°C unter Rühren;
c) Optionale Zugabe eines Alkali- oder Erdalkalihalogenids zur Fällung überschüssiger Silberionen;
d) Einfache oder mehrfache Filtration zur Abtrennung von unlöslichen Silberhalogeniden;
e) Fällung bis des Reaktionsproduktes;
f) Abfiltrieren des Reaktionsproduktes, um das feste Reaktionsprodukt und das Filtrat zu erhalten und optionales Waschen des Reaktionsproduktes.

3. Verfahren nach Anspruch 2, wobei die Fällung in Schritt e) durch Abdestillieren von Wasser unter reduziertem Druck bei einer Temperatur von unter 50°C, durch Zugabe eines Fällungsmittels oder deren Kombinationen bewirkt wird.

4. Verfahren nach Anspruch 3, wobei etwa 75% bis etwa 65% des Wassers abdestilliert wird.

5. Verfahren nach Anspruch 3, wobei das Fällungsmittel ein organisches Lösemittel ist, insbesondere Ethanol oder Aceton.

6. Verfahren nach einem oder mehreren der Ansprüche 2 bis 5, wobei Schritt c) einen Fällungsschritt zur Fällung überschüssiger Erdalkaliionen umfasst.

7. Verfahren nach einem oder mehreren der Ansprüche 2 bis 6, wobei das Silber-p-toluolsulfonat gegenüber dem cis-diamminodihalogenido Pt(II)-Komplex in der zweifachen molaren Menge eingesetzt wird.

8. Verfahren nach einem oder mehreren der Ansprüche 2 bis 7, enthaltend den weiteren Schritt g), in welchem das Filtrat auf eine Temperatur zwischen 0°C und 10°C gekühlt und ausgefallenes Reaktionsprodukt abfiltriert wird.

9. Verfahren nach einem oder mehreren der Ansprüche 2 bis 8, wobei in Schritt e) vor dem Fällen der pH-Wert durch Zugabe einer Säure auf einen pH-Wert von 1,5 oder niedriger, vorteilhaft von 1 oder niedriger eingestellt wird.

10. Verfahren nach einem oder mehreren der Ansprüche 2 bis 9, wobei das Verfahren unter Lichtausschluß, Sauerstoffausschluß oder deren Kombinationen durchgeführt wird.

11. Verfahren zur Herstellung einer Zusammensetzung enthaltend Wasser, cis-(diammino-diaquo)platin di-(p-toluolsulfonat) und optionale weiteren Substanzen, wobei cis-(diammino-diaquo)platin di-(p-toluolsulfonat) nach einem oder mehreren der Ansprüche 2 bis 10 hergestellt und in Wasser oder einer wässrigen Lösung aufgelöst wird.

12. cis-(diammino-diaquo)platin di-(p-toluolsulfonat) nach Anspruch 1, erhältlich nach einem oder mehreren der Ansprüche 2 bis 11.

13. Verwendung von cis-(diammino-diaquo)platin bis-(p-toluolsulfonat) nach Anspruch 1 oder dessen Zubereitungen als Analysenstandard.

14. Analysenkit enthaltend cis-(diammino-diaquo)platin di-(p-toluolsulfonat) nach Anspruch 1 oder cis-(diammino-diaquo)platin bis-(p-toluolsulfonat) erhältlich nach einem oder mehreren der Ansprüche 2 bis 10 oder deren Zubereitungen.

15. Verwendung der Verbindung nach Anspruch 1 als Precursor für die Synthese von cytostatisch aktiven Platinkomplexen.

16. Verbindung nach Anspruch 1 zur Verwendung als Aktivsubstanz in Cytostatika.

## Claims

1. The compound, cis-[(NH₃)₂Pt(H₂O)₂](O-Tos)₂, cis-(diammine-diaqua)platinum di-(p-toluene sulfonate), of the formula, 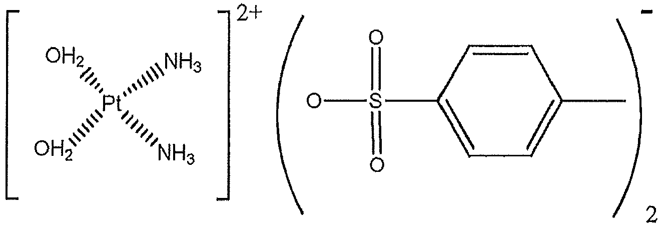 and polymers thereof.

2. Method for the preparation of the compound according to claim 1, **characterized by** the following steps:
a) providing a specific amount of a cis-diammine dihalide Pt (II) complex in an aqueous solution;
b) reacting this cis-diammine dihalide Pt (II) complex with the 1.8- to 2.2-fold molar amount (with respect to the cis-diammine dihalide Pt (II) complex) of silver-p-toluene sulfonate at a temperature of less than 100 °C while stirring;
c) optional addition of an alkaline or alkaline earth halide to precipitate excess silver ions;
d) single or multiple filtration for the separation of insoluble silver halides;
e) precipitation of the reaction product;
f) filtration of the reaction product in order to obtain the solid reaction product and the filtrate, and an optional washing of the reaction product.

3. Method according to claim 2, wherein the precipitation in step e) is accomplished by distilling off water under reduced pressure at a temperature below 50 °C, by addition of a precipitant or combinations thereof.

4. Method according to claim 3, wherein about 75% to about 65% of the water is distilled off.

5. Method according to claim 3, wherein the precipitant is an organic solvent - in particular, ethanol or acetone.

6. Method according to one or more of claims 2 through 5, wherein step c) comprises a precipitation step for the precipitation of excess alkaline earth ions.

7. Method according to one or more of claims 2 through 6, wherein the silver-p-toluene sulfonate, in comparison to the cis-diamminedihalide Pt (II) complex, is used in the doubled molar amount.

8. Method according to one or more of claims 2 through 7, including the additional step g), in which the filtrate is cooled to a temperature between 0 °C and 10 °C, and the precipitated reaction product is filtered off.

9. Method according to one or more of claims 2 through 8, wherein, in step e), prior to the precipitation, the pH value is adjusted to a pH value of 1.5 or less - advantageously, of 1 or less - by addition of an acid.

10. Method according to one or more of claims 2 through 9, wherein the method is carried out in the absence of light, absence of oxygen, or combinations thereof.

11. Method for producing a composition containing water, cis-(diammine-diaqua)platinum di-(p-toluene sulfonate), and optional further substances, wherein cis-(diammine-diaqua)platinum di-(p-toluene sulfonate) according to one or more of claims 2 through 10 is produced and dissolved in water or an aqueous solution.

12. cis-(diammine-diaqua)platinum di-(p-toluene sulfonate) according to claim 1, obtainable according to one or more of claims 2 through 11.

13. Use of cis-(diammine-diaqua)platinum bis-(p-toluene sulfonate) according to claim 1 or preparations thereof as an analytical standard.

14. Analysis kit containing cis-(diammine-diaqua)platinum di-(p-toluene sulfonate) according to claim 1 or cis-(diammine-diaqua)platinum bis-(p-toluene sulfonate) obtainable according to one or more of claims 2 through 10, or preparations thereof.

15. Use of the compound according to claim 1 as a precursor for the synthesis of cytostatically-active platinum complexes.

16. Compound according to claim 1 for use as an active substance in cytostatics.

## Revendications

1. Composé cis-[(NH₃)₂Pt(H₂O)₂](O-Tos)₂, di-(p-toluènesulfonate) de cis-(diamino-diaquo)platine de formule 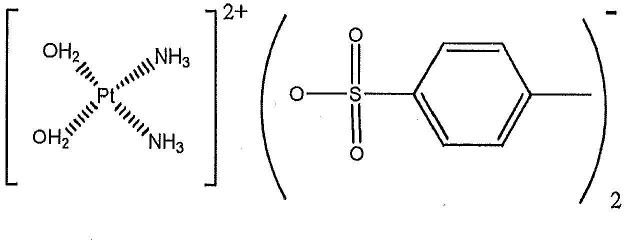 et ses polymères.

2. Procédé pour la préparation du composé selon la revendication 1, **caractérisé par** les étapes suivantes :
a) préparation d'une quantité déterminée d'un complexe cis-diaminodihalogéno-Pt (II) en solution aqueuse ;
b) transformation de ce complexe cis-diaminodihalogéno-Pt (II) avec 1,8 à 2,2 fois la quantité molaire (par rapport au complexe cis-diaminodihalogéno-Pt (II)) de p-toluènesulfonate d'argent à une température inférieure à 100 °C sous agitation ;
c) addition facultative d'un halogénure de métal alcalin ou alcalino-terreux pour la précipitation des ions d'argent en excès ;
d) filtration unique ou multiple pour la séparation des halogénures d'argent insolubles ;
e) précipitation du produit de réaction ;
f) séparation par filtration du produit de réaction pour obtenir le produit de réaction solide et le filtrat et lavage facultatif du produit de réaction.

3. Procédé selon la revendication 2, la précipitation dans l'étape e) étant provoquée par élimination par distillation d'eau sous pression réduite à une température inférieure à 50 °C, par addition d'un agent de précipitation ou par leur combinaison.

4. Procédé selon la revendication 3, environ 75 % à environ 65 % de l'eau étant éliminés par distillation.

5. Procédé selon la revendication 3, l'agent de précipitation étant un solvant organique, en particulier l'éthanol ou l'acétone.

6. Procédé selon l'une ou plusieurs des revendications 2 à 5, l'étape c) comprenant une étape de précipitation pour la précipitation d'ions de métal alcalino-terreux en excès.

7. Procédé selon l'une ou plusieurs des revendications 2 à 6, le p-toluènesulfonate d'argent étant utilisé en une quantité molaire double par rapport au complexe cis-diaminodihalogéno-Pt (II).

8. Procédé selon l'une ou plusieurs des revendications 2 à 7, contenant l'étape supplémentaire g), dans laquelle le filtrat est refroidi à une température entre 0 °C et 10 °C et le produit de réaction précipité est éliminé par filtration.

9. Procédé selon l'une ou plusieurs des revendications 2 à 8, le pH dans l'étape e), avant la précipitation, étant réglé à un pH de 1,5 ou moins, avantageusement de 1 ou moins, par addition d'un acide.

10. Procédé selon l'une ou plusieurs des revendications 2 à 9, le procédé étant réalisé à l'abri de la lumière, à l'abri de l'oxygène ou leur combinaison.

11. Procédé pour la préparation d'une composition contenant de l'eau, du di-(p-toluènesulfonate) de cis-(diamino-diaquo)platine et d'autres substances facultatives, le di-(p-toluènesulfonate) de cis-(diamino-diaquo)platine étant préparé selon l'une ou plusieurs des revendications 2 à 10 et dissous dans de l'eau ou dans une solution aqueuse.

12. Di-(p-toluènesulfonate) de cis-(diamino-diaquo)platine selon la revendication 1, pouvant être obtenu selon l'une ou plusieurs des revendications 2 à 11.

13. Utilisation du bis-(p-toluènesulfonate) de cis-(diamino-diaquo)platine selon la revendication 1 ou de ses préparations comme témoin d'analyse.

14. Kit d'analyse contenant du di-(p-toluènesulfonate) de cis-(diamino-diaquo)platine selon la revendication 1 ou du di-(p-toluènesulfonate) de cis-(diamino-diaquo)platine pouvant être obtenu selon l'une ou plusieurs des revendications 2 à 10 ou leurs préparations.

15. Utilisation du composé selon la revendication 1 comme précurseur pour la synthèse de complexes du platine, actifs d'un point de vue cytostatique.

16. Composé selon la revendication 1 pour une utilisation comme substance active dans des agents cytostatiques.
